**Europäisches Patentamt**

**European Patent Office**

**Office européen des brevets**

(19)

(11) Numéro de publication : **0 054 012**
**B1**

(12) **FASCICULE DE BREVET EUROPÉEN**

(45) Date de publication du fascicule du brevet :
20.08.86

(51) Int. Cl.⁴ : **G 01 N  1/28**, G 01 N 21/67,
G 01 N 33/20

(21) Numéro de dépôt : **81870040.3**

(22) Date de dépôt : **08.10.81**

(54) **Procédé de traitement d'un échantillon d'acier.**

(30) Priorité : **10.10.80 BE 202415**

(43) Date de publication de la demande :
**16.06.82 Bulletin 82/24**

(45) Mention de la délivrance du brevet :
**20.08.86 Bulletin 86/34**

(84) Etats contractants désignés :
**AT CH DE FR GB IT LI LU NL SE**

(56) Documents cités :
**BE-A- 885 642**
**DE-B- 1 598 580**
**APPLIED SPECTROSCOPY, vol. 25, no. 3, 1971, BALTI-MORE (US), J.P. CUMMINGS et al., "Spectrographic analysis of cast iron using fused buttons", pages 342-344**
**FRESENIUS ZEITSCHRIFT FÜR ANALYTISCHE CHE-MIE, vol. 257, no. 4, 1971, BERLIN (DE), K. OHLS et al. "Fortschritte bei der emissionsspektrometrischen Stahlanalyse; IV. Untersuchung einiger Matrixef-fekte", pages 257-264**

(73) Titulaire : **COCKERILL SAMBRE**
**Quai Greiner, 1**
**B-4100 Seraing (BE)**

(72) Inventeur : **Humbert, Maurice Emile**
**rue Heid du Moulin, 29**
**B-4051 Plainevaux (BE)**

(74) Mandataire : **Vanderperre, Robert et al**
**Bureau VANDER HAEGHEN 63 Avenue de la Toison d'Or**
**B-1060 Bruxelles (BE)**

# Description

La présente invention concerne un procédé de traitement d'un échantillon d'acier afin de permettre la détermination rapide de la teneur en aluminium soluble avec une grande fiabilité.

Il est important pour le sidérurgiste de connaître de façon précise la teneur en aluminium de l'acier produit, aussi bien l'aluminium métallique que l'aluminium soluble.

La détermination de la composition chimique de l'acier liquide peut évidemment être effectuée par analyse chimique et ce procédé est d'ailleurs couramment utilisé dans l'industrie sidérurgique. Ce procédé est cependant lent et ne s'adapte guère à la production d'acier en coulée continue de sorte que l'on fait alors appel à un procédé d'analyse spectrale à lecture directe plus rapide. Un tel procédé consiste à soumettre l'échantillon d'acier à une série d'impulsions d'étincelles et à mesurer les impulsions de courant photoélectriques produites à l'aide d'un spectromètre. La méthode de mesure classique donne la teneur totale en aluminium et consiste à intégrer les impulsions photoélectriques sur un certain laps de temps. Un perfectionnement à cette méthode, appelé méthode d'analyse par distribution d'impulsions (P.D.A.) consiste à mesurer l'intensité spectrale pour chaque impulsion d'étincelle. Cette méthode spécialement destinée à la détermination de la teneur d'aluminium soluble de l'acier nécessite un appareillage électronique coûteux pour l'intégration, la mémorisation et la distribution des impulsions photoélectriques. De plus, des échantillons d'acier produits par la déposante et soumis à la fois à des essais d'analyse chimique et à des essais d'analyse spectrale suivant la méthode P.D.A. ont révélé une divergence irrégulière entre les résultats d'analyse chimique et les résultats d'analyse spectrale en ce qui concerne la teneur en aluminium soluble pour des échantillons contenant une forte teneur en alumine.

Un perfectionnement aux procédés d'analyse spectrale consiste à effectuer la fusion locale par arc électrique dans une enceinte sous atmosphère inerte. Cet art antérieur est illustré par exemple par les publications DE-B-1 598 580, Fresenius Zeitschrift für Analytische Chemie, volume 257, n° 4, 1971, Berlin (DE), pages 257 à 264 et Applied Spectroscopy, volume 25, n° 3, 1971, Baltimore (US), pages 342 à 344.

Suivant l'art antérieur, la fusion locale par l'arc électrique se fait sans modification structurale des zones soumises à la fusion et l'analyse spectrale s'effectue sur les zones soumises à la fusion locale, c'est-à-dire des zones contenant des inclusions. Les résultats de l'analyse dans ce cas ne sont significatifs, comme l'a constaté le demandeur, que lorsque la composition de l'échantillon est homogène mais les résultats sont faussés lorsque l'acier contient des concentrations locales de certains éléments, par exemple des inclusions d'alumine dans le cas où l'on désire faire le dosage de l'aluminium dans l'acier.

Le demandeur a cherché à rendre plus fiables les mesures d'analyse spectrale effectuées sur des échantillons prélevés sur le site de coulée d'acier afin d'améliorer la fiabilité du dosage de l'aluminium soluble des aciers, et donc la qualité de ces aciers, même chargés en alumine.

Ce but est atteint par le procédé de traitement d'un échantillon d'acier pour analyse spectrale selon l'invention, qui vise à produire une zone locale d'acier purifié affleurant à la surface d'une face de l'échantillon. Ce procédé consiste à soumettre d'abord l'échantillon à un écroûtage métallique rapide pour éliminer les oxydes superficiels, puis il est placé sur un support métallique refroidi. Ensuite, l'échantillon est soumis à une fusion locale à l'arc électrique dans une enceinte où est maintenue une atmosphère inerte, l'arc électrique étant maintenu pendant un laps de temps suffisant pour que les inclusions localisées dans la zone avoisinant la zone d'impact de l'arc électrique se trouvent rassemblées dans la zone soumise à la fusion, mais insuffisant pour que l'échantillon soit percé, et on effectue un surfaçage rapide de la face de l'échantillon ayant subi la fusion locale de manière à éliminer la zone dans laquelle sont rassemblées les inclusions et produire ainsi une zone d'acier purifié à la surface de l'échantillon.

L'échantillon ainsi préparé et purifié, peut être soumis à l'analyse spectrale classique sans appareillage électronique supplémentaire pour le traitement des impulsions.

L'avantage du procédé de traitement suivant l'invention est qu'il permet une analyse spectrale rapide des échantillons d'acier, qui est plus économique que le procédé d'analyse par distribution d'impulsions et qui permet d'obtenir directement la teneur en aluminium métallique ou soluble car ce procédé a pour effet d'éliminer les inclusions d'alumine de la zone où se produit l'étincelage.

L'invention est exposée plus en détail dans ce qui suit en se référant aux dessins ci-annexés sur lesquels :

la figure 1 montre en coupe un échantillon d'acier sortant de production ;

la figure 2 montre en coupe l'échantillon de la figure 1 après fusion locale suivant le procédé de l'invention ;

la figure 3 montre en coupe l'échantillon de la figure 2 après la phase de surfaçage du procédé suivant l'invention ;

la figure 4 représente schématiquement un mode d'exécution d'une installation pour le traitement d'échantillons d'acier suivant l'invention.

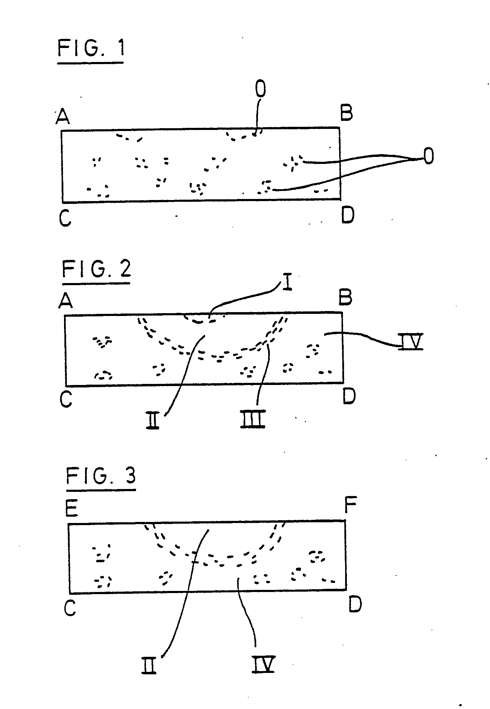

Le procédé suivant l'invention s'applique à un échantillon d'acier prélevé à sa sortie du four. Le traitement de l'échantillon commence par un écroûtage métallique rapide visant à éliminer les oxydes superficiels. L'échantillon contient, noyées dans l'acier, des inclusions d'aluminium soluble, notamment des inclusions d'aluminium soluble, notamment des inclusions d'alumine. Sur la figure 1 on a représenté un échantillon d'acier 10 dans lequel des inclusions de composés d'aluminium sont repérées par la lettre O.

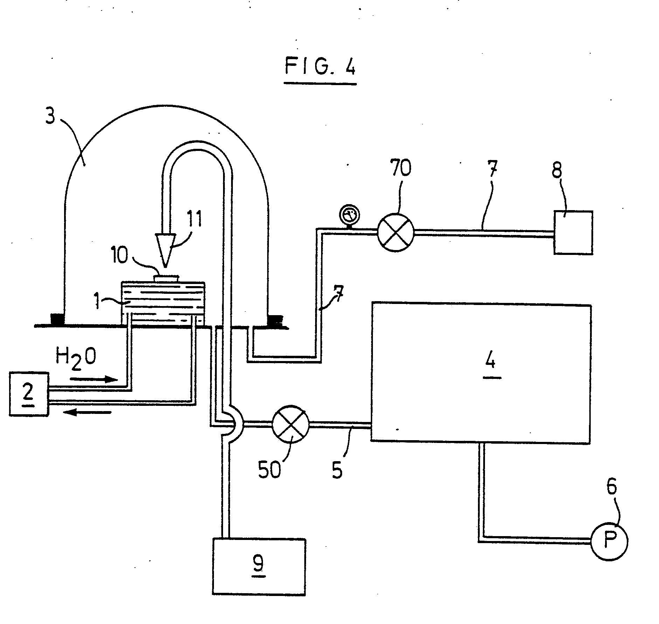

Se référant à la figure 4, l'échantillon écroûté 10 est alors placé sur un support métallique 1 refroidi par une circulation d'eau débitée par une source d'alimentation 2. Le support 1 se trouve placé à l'intérieur d'une enceinte 3 dans laquelle est maintenue une atmosphère inerte. A cet effet, le volume de l'enceinte 3 se trouve en communication avec un réservoir 4 par le conduit 5 et le réservoir 4 se trouve raccordé à une pompe à vide 6. Dans le volume de l'enceinte 3 débouche également une conduite 7 d'alimentation de gaz inerte de grande pureté, par exemple de l'argon provenant d'un réservoir 8. Sur les conduites 5 et 7 sont branchées des vannes 50 et 70. Après avoir fait le vide d'air dans l'enceinte 3, le gaz inerte est introduit sous pression, par exemple une pression de 400 mm. Pour assurer un brusque vide d'air, le réservoir 4 a avantageusement un volume égal à plusieurs fois le volume de l'enceinte 3. Ce réservoir n'est pas représenté à l'échelle sur la figure 4.

A l'aide d'une électrode 11 raccordée à un bloc de soudure 9 et disposée au-dessus de l'échantillon 10 sur le support, on provoque une fusion locale par arc électrique pendant un laps de temps prédéterminé, fonction de l'épaisseur de l'échantillon. Pour un échantillon de quelque 10 à 15 mm d'épaisseur, la durée de l'arc peut être de l'ordre de 15 à 60 secondes. Il se produit alors dans l'échantillage 10 une zone dans laquelle se rassemblent les inclusions de la zone fondue, laissant celle-ci à l'état pur. La figure 2 montre la formation de la zone de rassemblement des inclusions I. Cette zone est entourée d'une zone II purifiée par la fusion. La zone II est une zone qui reste pâteuse au cours de la fusion et la zone IV est une zone non influencée par la fusion. La durée de la phase de fusion doit être suffisamment longue pour réaliser une purification locale de la zone II mais pas trop longue cependant pour éviter un percement de l'échantillon.

Le traitement de l'échantillon se termine par un surfaçage rapide de la face ayant subi la fusion locale afin d'éliminer la zone I contenant les inclusions. La figure 3 montre l'échantillon 10 après cette opération.

L'échantillon ayant ainsi été traité est alors prêt pour une analyse spectrale de la zone purifiée.

Des mesures effectuées sur un grand nombre d'échantillons d'acier ont donné des teneurs en aluminium soluble des échantillons traités suivant l'invention qui correspondent avec une bonne précision aux teneurs déterminées par analyse chimique.

## Revendications

1. Procédé de traitement d'un échantillon d'acier devant subir une analyse spectrale, afin de produire une zone locale d'acier purifié affleurant à la surface d'une face de l'échantillon, procédé selon lequel l'échantillon refroidi est soumis à l'action d'un arc électrique produit entre une électrode (11) et une face de l'échantillon (10) dans une enceinte (3) où est maintenue une atmosphère inerte, caractérisé en ce qu'il comporte les étapes suivantes :

— l'échantillon est d'abord soumis à un écroûtage métallique rapide pour éliminer les oxydes superficiels, puis est placé sur un support (1) métallique refroidi,

— l'échantillon est soumis à une fusion locale à l'arc électrique, celui-ci étant maintenu pendant un laps de temps suffisant pour que les inclusions (0) localisées dans la zone (II) avoisinant la zone d'impact de l'arc électrique se trouvent rassemblées dans la zone (I) soumise à la fusion mais insuffisant pour que l'échantillon soit percé,

— on effectue un surfaçage rapide de la face de l'échantillon ayant subi la fusion locale de manière à éliminer la zone (I) dans laquelle sont rassemblées les inclusions et produire ainsi une zone d'acier purifié (II) à la surface de l'échantillon.

2. Procédé selon la revendication 1, caractérisé en ce que le temps d'application de l'arc électrique entre l'électrode et la face de l'échantillon est compris entre 15 et 60 secondes environ.

## Claims

1. A method of preparing a steel sample for spectrographic analysis in order to produce a local zone of purified steel flush with the surface of a face of said sample, wherein the sample is subjected to an electric arc established between an electrode (11) and a face of the sample (10) within an enclosure (3) in which an inert atmosphere is maintained, characterized by the following steps :

the sample is first subjected to a rapid metal crust removal operation thereby to remove the superficial oxides, and then placed on a cooled metal support (1),

the sample is subjected to a local melting using an electric arc, said local melting being maintained during a sufficient time interval such that the inclusions (0) which are contained in the zone (II) adjacent to the impact area of the electric arc are gathered within the zone (I) subjected to the local melting but said time interval being insufficient for the sample being pierced,

applying a rapid surfacing of the sample face which was subjected to said local melting thereby to remove said zone (I) within which the inclusions have been gathered and to produce a zone of purified steel (II) flush with the surface of the sample.

2. A method according to claim 1, characterized in that the application time of the electric arc between the electrode and the sample face is comprised between 15 and 60 seconds approximately.

## Patentansprüche

1. Verfahren zur Aufbereitung einer einer Spektralanalyse zu unterwerfenden Stahlprobe, zwecks Bildung einer versenkten lokalen Zone gereinigten Stahls an einer Fläche der Probe, bei welchem Verfahren die gekühlte Probe der Wirkung eines Lichtbogens ausgesetzt wird, der in einem umschlossenen Raum (3) in dem eine inerte Atmosphäre aufrechterhalten wird, zwischen einer Elektrode (11) und einer Fläche der Probe (10) erzeugt wird, dadurch gekennzeichnet, daß es folgende Schritte umfaßt :

die Probe wird zunächst einem raschen Metallschälvorgang unterworfen, um die Oberflächenoxide zu beseitigen, und wird dann auf einem gekühlten metallischen Träger (1) angeordnet,

die Probe wird mittels des Lichtbogens lokal erschmolzen, wobei der Lichtbogen während einer Zeitspanne aufrechterhalten wird, die ausreicht, daß die in der an die Zone des Auftreffens des Lichtbogens angrenzenden Zone (II) lokalisierten Einschlüsse (0) in der dem Schmelzvorgang unterworfenen Zone (I) gesammelt werden, jedoch nicht dazu ausreicht, daß die Probe durchgebrannt wird,

man nimmt eine rasche Oberflächenbehandlung jener Fläche der Probe, welche dem lokalen Schmelzvorgang unterworfen wurde, vor, solcherart, daß die Zone (I), in der die Einschlüsse gesammelt sind, entfernt und damit auf der Oberfläche der Probe eine Zone (II) gereinigten Stahls erzeugt wird.

2. Verfahren nach Anspruch 1, dadurch gekennzeichnet, daß die Dauer der Anwendung des Lichtbogens zwischen der Elektrode und der Fläche der Probe etwa zwischen 15 und 60 s beträgt.

0 054 012

FIG. 1

FIG. 2

FIG. 3

FIG. 4